# FASCICULE DE BREVET EUROPEEN

(11) **EP 1 291 008 B1**
(45) Date de publication et mention de la délivrance du brevet: **15.02.2006**
(21) Numéro de dépôt: 02291957.5
(22) Date de dépôt: 02.08.2002
(51) Int. Cl.: A61K 8/34, A61K 8/37, A61K 8/35, A61K 8/49, A61K 8/81, A61Q 17/04

(54) **Composition filtrante contenant au moins un dérivé de dibenzoylméthane et le 1,1,3,-tri-(2-méthyl-4-hydroxy-5-tert-butylphenyl)-butane**
Mindestens ein Dibenzoylmethanderivat und 1,1,3,-Tri-(2-methyl-4-hydroxy-5-tert-butylphenyl)-butan enthaltende Filterzusammensetzung
Solar composition containing at least one dibenzoylmethane derivative and 1,1,3,-tri-(2-methyl-4-hydroxy-5-tert-butylphenyl)-butane

(30) Priorité: 27.08.2001 FR 0111139
(43) Date de publication de la demande: 12.03.2003
(73) Titulaire: L'OREAL, 75008 Paris (FR)
(72) Inventeur: Candau, Didier, 91570 Bievres (FR); Aubert, Fabien, 75018 Paris (FR)
(74) Mandataire: Miszputen, Laurent

(56) Documents cités:
- FR-A- 2 384 842
- M.-C. MARTINI ET AL.: "actifs et adiitifs en cosmetologie" 1992 , LAVOISIER TEC & DOC , PARIS XP002203973 * page 190 - page 195 *
- G. MARGINEAN LAZAR ET AL.: "indoor/outdoor sunscreens evaluation by the stripping method" PROCEEDINGS OF THE INTERNATIONAL SYMPOSIUM ON CONTROLLED RELEASE OF BIOACTIVE MATERIALS, 24, 1997, pages 917-918, XP001074039

## Description

L'invention se rapporte à une composition cosmétique ou dermatologique, à usage topique, en particulier pour la photoprotection de la peau et des cheveux,
caractérisée par le fait qu'elle comprend au moins dans un support cosmétiquement acceptable au moins :
(a) un filtre UV du type dérivé du dibenzoylméthane et
(b) le 1,1,3-tri-(2-méthyl-4-hydroxy-5-tert-butylphenyl)-butane.

L'invention se rapporte également à un procédé pour améliorer la stabilité d'au moins un dérivé du dibenzoylméthane vis-à-vis du rayonnement UV consistant à associer au dit dérivé de dibenzoylméthane une quantité efficace de 1,1,3-tri-(2-méthyl-4-hydroxy-5-tert-butylphenyl)-butane.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la Société HOFFMANN LA ROCHE.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

Les dérivés de 1,3,5-triazine sont particulièrement recherchés dans la cosmétique solaire du fait qu'ils sont fortement actifs dans l' UVB et même dans l'UV-A pour certains de ces composés selon la nature des substituants mis en jeu. Ils sont notamment décrits dans les demandes de brevet US 4367390, EP863145, EP517104, EP570838, EP507691, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCI), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]
- 1,3,5-triazine ou « Diethylhexyl Butamido Triazone » (nom INCI) vendu sous le nom commercial « UVASORB HEB » par SIGMA 3V,

Toutefois, la Demanderesse a constaté que ces dérivés de 1,3,5-triazine, lorsqu'ils sont en présence de dérivés du dibenzoylméthane, en particulier du 4-tert-butyl-4'-méthoxydibenzoylméthane, et sous irradiation UV, présentent l'inconvénient de se dégrader chimiquement de façon importante. Dans ces conditions, l'association des deux filtres ne permet plus une protection solaire large prolongée de la peau et des cheveux.
La Demanderesse vient maintenant de découvrir, de façon surprenante, qu'en associant aux dérivés du dibenzoylméthane mentionnés ci-dessus une quantité efficace de 1,1,3-tri-(2-méthyl-4-hydroxy-5-tert-butylphenyl)-butane, il était possible d'améliorer de manière substantielle et remarquable, la stabilité photochimique (ou photostabilité) de ces mêmes dérivés du dibenzoylméthane.De plus, la Demanderesse a également découvert que l'introduction du 1,1,1-tri-(2-méthyl-4-hydroxy-5-tert-butylphenyl)-butane dans une composition contenant l'association d'un dérivé de dibenzoylméthane, avec au moins un dérivé de 1,3,5-triazine permettait d'améliorer de façon tout à fait remarquable la photostabilité de ce dérivé de 1,3,5-triazine au sein de telles compositions, et donc l'efficacité globale de ces compositions.

Ainsi, conformément à l'un des objets de la présente invention, il est maintenant proposé une composition cosmétique ou dermatologique, à usage topique, caractérisée par le fait qu'elle comprend au moins, dans un support cosmétiquement acceptable :
(a) au moins un filtre UV du type dérivé du dibenzoylméthane et
(b) une quantité efficace de 1,1,3-tri-(2-méthyl-4-hydroxy-5-tert-butylphenyl)-butane.

La présente invention a également pour objet un procédé pour améliorer la stabilité d'au moins un dérivé du dibenzoylméthane vis-à-vis du rayonnement UV consistant à associer au dit dérivé de dibenzoylméthane une quantité efficace de 1,1,3-tri-(2-méthyl-4-hydroxy-5-tert-butylphenyl)-butane.

Un autre objet de la présente invention consiste en une composition cosmétique ou dermatologique, à usage topique, caractérisée par le fait qu'elle comprend au moins, dans un support cosmétiquement acceptable :
( ) au moins un filtre UV du type dérivé du dibenzoylméthane et
( ) au moins un filtre UV du type dérivé de 1,3,5-triazine et
( ) une quantité efficace de 1,1,3-tri-(2-méthyl-4-hydroxy-5-tert-butylphenyl)-butane.

La présente invention a également pour objet un procédé pour améliorer la stabilité d'au moins un dérivé de 1,3,5-triazine en présence d'au moins un dérivé du dibenzoylméthane vis-à-vis du rayonnement UV consistant à ajouter une quantité efficace de 1,1,3-tri-(2-méthyl-4-hydroxy-5-tert-butylphenyl)-butane.

La présente invention a également enfin pour objet l'utilisation du 1,1,3-tri-(2-méthyl-4-hydroxy-5-tert-butylphenyl)-butane dans la préparation d'une composition cosmétique ou dermatologique comprenant au moins un dérivé du dibenzoylméthane dans le but de d'améliorer la stabilité vis-à-vis des rayons UV dudit dérivé de dibenzoylméthane contenu.

La présente invention a également enfin pour objet l'utilisation du 1,1,3-tri-(2-méthyl-4-hydroxy-5-tert-butylphenyl)-butane dans la préparation d'une composition cosmétique ou dermatologique comprenant au moins un dérivé du dibenzoylméthane et au moins un dérivé de 1,3,5-triazine dans le but de d'améliorer la stabilité vis-à-vis des rayons UV dudit dérivé de 1,3,5-triazine contenu.

Par quantité efficace de 1,1,3-tri-(2-méthyl-4-hydroxy-5-tert-butylphenyl)-butane conforme à l'invention, on entend une quantité suffisante pour obtenir une amélioration notable et significative de la photostabilité du ou des dérivés du dibenzoylméthane (ou bien du ou des dérivés de1,3,5-triazine) de la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

D'autres caractéristiques, aspects et avantages de la présente invention apparaîtront à la lecture de la description détaillée qui va suivre.

Le composé 1,1,3-tri-(2-méthyl-4-hydroxy-5-tert-butylphenyl)-butane répond à la formule suivante :

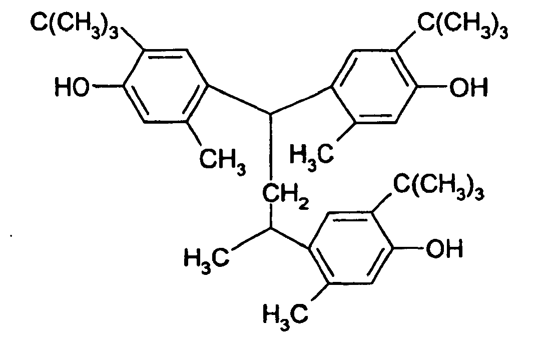

Le composé 1,1,3-tri-(2-méthyl-4-hydroxy-5-tert-butylphenyl)-butane est présent de préférence dans la composition de l'invention dans des proportions allant de 0,1 % à 15% en poids et plus préférentiellement de 0,2 à 10% en poids, par rapport au poids total de la composition.

Comme indiqué précédemment, les dérivés du dibenzoylméthane destinés à être photostabilisés dans le cadre de la présente invention sont des produits déjà bien connus en soi et décrits notamment dans les documents FR2326405, FR2440933 et EP0114607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société HOFFMANN LA ROCHE, ce filtre répondant donc à la formule développée suivante :

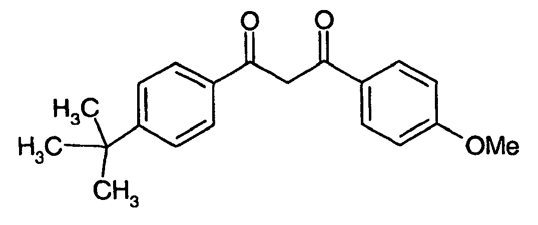

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

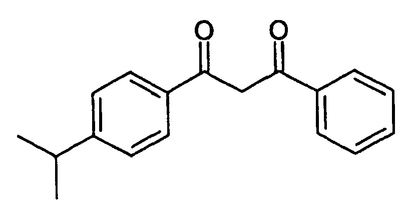

Le ou les dérivés du dibenzoylméthane peuvent être présents dans les compositions conformes à l'invention à des teneurs qui varient de préférence de 0,1 % à 15 % en poids et plus préférentiellement de 0,5 à 10% en poids, par rapport au poids total de la composition.

Parmi les dérivés de 1,3,5-triazine utilisables dans le cadre de la présente invention, on peut utiliser notamment ceux répondant à la formule (I) suivante : 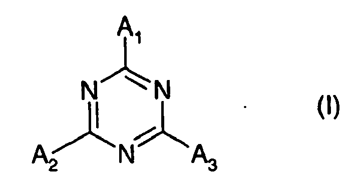
dans laquelle les radicaux A₁, A₂ et A₃, identiques ou différents sont choisis parmi les groupes de formules (II) : 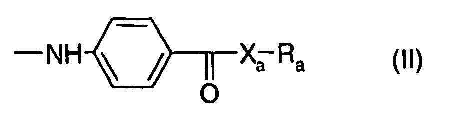
dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles
ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 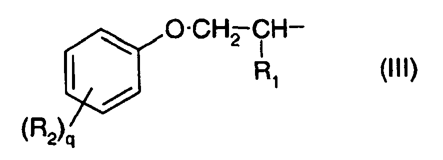 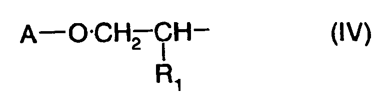 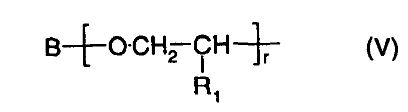
dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

Une première famille plus particulièrement préférée de dérivés de 1,3,5-triazine, notamment décrite dans le document EP-A-0 517 104, est celle des 1,3,5-triazines répondant à la formule (I) dans laquelle les A₁, A₂ et A₃ sont de formule (II) et présentent les caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles:
- B est un radical alkyle en C₁-C₄;
- R₂ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;

le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 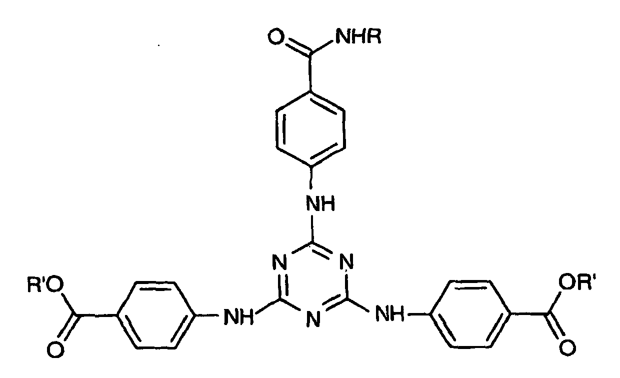
dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est celle des 1,3,5-triazines répondant à la formule (I) dans laquelle les A₁, A₂ et A₃ sont de formule (II) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 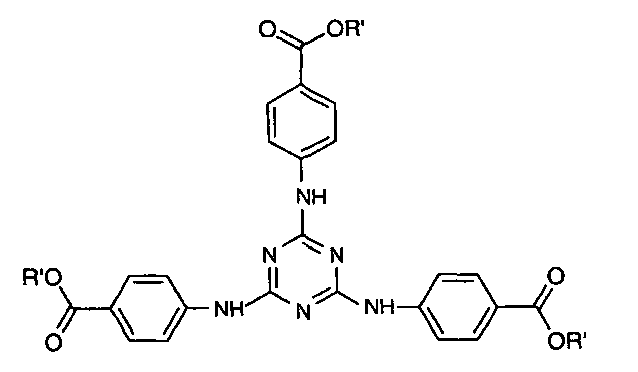
dans laquelle R' désigne un radical 2-éthyl hexyle.

Le ou les dérivés de 1,3,5-triazine sont généralement présents dans les compositions de l'invention à une teneur pouvant aller de 0,1 % à 15 %, de préférence de 0,5 % à 10 % en poids, par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés salicyliques, les dérivés du camphre ; les dérivés de la benzophénone ; les dérivés de β,β'-diphénylacrylate, les dérivés de benzotriazole ; les dérivés de benzalmalonate ; les dérivés de benzimidazole ; les dérivés de triazine autres que ceux de formule (I) cités précédemment; les imadazolines ; les dérivés bis-benzoazolyle tels que décrits dans les brevets EP669323 et US 2,463,264 ; les dérivés de l'acide p-aminobenzoïque (PABA) ; les dérivés de méthylène bis-(hydroxyphényl benzotriazole) tels que décrits dans les demandes US5,237,071, USS,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet EP0967200 et DE19755649 (faisant partie intégrante du contenu de la description).

Comme exemples de filtres organiques complémentaires actifs dans l'UV-A et/ou l'UV-B, on peut citer; désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicvliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12
- le 2-(4-diéthylamino-2-hydroxybenzoyl)-benzoate de n-hexyle

### Dérivés du benzylidène camphre :

- 4-Methylbenzylidene camphor vendu sous le nom Eusolex 6300 par MERCK
- 3-Benzylidene camphor fabriqué sous le nom « MEXORYL SD» par CHIMEX,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés de benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

### Dérivés de la triazine :

- Anisotriazine vendu sous le nom commercial «TINOSORB S » par CIBA SPECIALTY CHEMICALS
- la 2,4,6- tris-(4'amino-benzalmalonate de diisobutyle)-s-triazine.

### Dérivés de benzotriazole :

- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
- Drometrizole Trisiloxane vendu sous le nom « SILATRIZOLE » par RHODIA CHIMIE ,

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés de benzalmalonate :

- Polyorganosiloxane à fonction benzalmalonate vendu sous la dénomination commerciale « PARSOL SLX » par HOFFMANN LA ROCHE et leurs mélanges.

Les filtres UV organiques complémentaires plus particulièrement préférés sont choisis parmi les composés suivants :
- Ethylhexyl Salicylate,
- Octocrylene,
- Phenylbenzimidazole Sulfonic Acid,
- 4-Methylbenzylidene camphor,
- Disodium Phenyl Dibenzimidazole Tetra-sulfonate,
- Terephthalylidene Dicamphor Sulfonic,
- Ethylhexyl triazone
- Diethylhexyl Butamido Triazone
- la 2,4,6- tris-(4'amino-benzalmalonate de diisobutyle)-s-triazine
- Anisotriazine,
- Benzophenone-3,
- Benzophenone-4,
- Benzophenone-5,
- le 2-(4-diéthylamino-2-hydroxybenzoyl)-benzoate de n-hexyle
- Methylene bis-Benzotriazolyl Tetramethylbutylphenol,
- Drometrizole Trisiloxane

et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets

EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras, linéaires ou cycliques tels que les dérivés d'acide benzoïque, trimellitique et hydroxy-benzoïque Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la photostabilité, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Un autre objet de la présente invention réside dans un procédé pour améliorer la stabilité d'au moins un dérivé du dibenzoylméthane vis-à-vis du rayonnement UV consistant à associer au dit dérivé de dibenzoylméthane une quantité efficace de 1,1,3-tri-(2-méthyl-4-hydroxy-5-tert-butylphenyl)-butane tel que défini ci-dessus.

Un autre objet de la présente invention consiste en l'utilisation du 1,1,3-tri-(2-méthyl-4-hydroxy-5-tert-butylphenyl)-butane tel que défini ci-dessus dans la préparation d'une composition cosmétique ou dermatologique comprenant au moins un filtre UV du type dérivé du dibenzoylméthane dans le but de d'améliorer la stabilité vis-à-vis des rayons UV dudit dérivé du dibenzoylméthane.

Des exemples concrets, mais nullement limitatifs, illustrant l'invention, vont maintenant être donnés.

### Exemple 1

On étudie la photostabilité du dérivé de dibenzoylméthane sur plusieurs formulations dans lesquelles on fait varier la teneur en 1,1,1-tri-(2-méthyl-4-hydroxy-5-tert-butylphenyl)-butane (X). Le support commun à ces formulations est le suivant :

| **Support 1** | **% en poids** |
|---|---|
| Benzoate d'alcools en C12/C15 (WITCONOL TN -WITCO) | 15,00 |
| Butyl Methoxydibenzoylmethane (PARSOL 1789 - HOFFMANN LA ROCHE) | 2 |
| 1,1,3-tri-(2-méthyl-4-hydroxy-5-tert-butylphenyl)-butane (MIXXIM AO-30-FAIRMOUNT CHEMICAL) | x |
| Copolymère acide acrylique/itaconate de monostéaryl oxyéthyléné (200 OE) ( STRUCTURE 2001 - NATIONAL STARCH) | 3,33 |
| Triéthanolamine | 0,45 |
| Conservateurs | 1,20 |
| Eau démineralisée qsp | 100 |

### Méthode de mesure

Pour chaque formule, on a préparé 3 échantillons tests et 3 échantillons témoins . On a déposé, à la spatule, 2 mg/cm² de formule sur des plaques de polyméthacrylate de méthyle. Les plaques tests ont été exposées 1h 39 mn au SUN TEST HERAUS muni d'une lampe Xénon ayant comme éclairement UV-A : 3,02. 10-3 W/cm² et éclairement UV-B:2,1.10-4 W/cm² et les plaques témoins sont conservées pendant le même temps et à la même température (38-40°C) à l'obscurité. A l'issue de ce temps, on a procédé à l'extraction des filtres en immergeant chaque plaque dans 50 g d'éthanol et en les passant aux ultrasons pendant 15 mn pour assurer une bonne extraction . Les solutions obtenues sont analysées par spectrospectométrie UV. Pour chaque formule testée, le taux de butyl-méthoxy-dibenzoylméthane résiduel après exposition est donné dans le rapport de sa densité optique (DO) dans l'échantillon exposé à sa densité optique (DO) non exposé. On se place au maximum d'absorption correspondant au butyl-méthoxy-dibenzoylméthane : λₘₐₓ = 358 nm.

Les résultats obtenus sont résumés dans le tableau 1 suivant :

| x (%en poids) | DO résiduelle à 358 nm après 1 heure d'irradiation UVA |
|---|---|
| 0 | 11 ± 1 % |
| 0.5 | 21 ± 1 % |
| 1 | 37 ± 1 % |
| 2 | 60 ± 2% |

### Exemple 2

On étudie la photostabilité de l'octyltriazone en présence du butylméthoxydibenzoylméthane sur plusieurs formulations dans lesquelles on fait varier la teneur en 1,1,1-tri-(2-méthyl-4-hydroxy-5-tert-butylphenyl)-butane (X). Le support commun à ces formulations est le suivant :

| **Support 2** | **% en poids** |
|---|---|
| Benzoate d'alcools en C12/C15 (WITCONOL TN -WITCO) | 15,00 |
| Butyl Methoxydibenzoylmethane (PARSOL 1789 - HOFFMANN LA ROCHE) | 0,5 |
| 1,1,3-tri-(2-méthyl-4-hydroxy-5-tert-butylphenyl)-butane (MIXXIM AO-30-FAIRMOUNT CHEMICAL) | X |
| Octyltriazone (UVINUL T150- BASF) | 1,5 |
| Copolymère acide acrylique/itaconate de monostéaryl oxyéthyléné (200 OE) (STRUCTURE 2001 - NATIONAL STARCH) | 3,33 |
| Triéthanolamine | 0,45 |
| Conservateurs | 1,20 |
| Eau démineralisée qsp | 100 |

### Méthode de mesure

Pour chaque formule, on a préparé 3 échantillons tests et 3 échantillons témoins. On a déposé, à la spatule, 2 mg/cm² de formule sur des plaques de polyméthacrylate de méthyle. Les plaques tests ont été exposées 1h 39 mn au SUN TEST HERAUS muni d'une lampe Xénon ayant comme éclairement UV-A : 3,02. 10-3 W/cm² et éclairement UV-B :2,1.10-4 W/cm² et les plaques témoins sont conservées pendant le même temps et à la même température (38-40°C) à l'obscurité. A l'issue de ce temps, on a procédé à l'extraction des filtres en immergeant chaque plaque dans 50 g d'éthanol et en les passant aux ultrasons pendant 15 mn pour assurer une bonne extraction . Les solutions obtenues sont analysées par spectrospectométrie UV. Pour chaque formule testée, le taux de butyl-méthoxy-dibenzoylméthane résiduel et le taux résiduel d'octyltriazone après exposition est donné dans le rapport de chaque densité optique respective (DO) dans l'échantillon exposé à la densité optique (DO) non exposé correspondant à chaque filtre. On se place au maximum d'absorption correspondant à chaque filtre :
Butylméthoxydibenzoylméthane : λₘₐₓ = 358 nm
Octyltriazone : λₘₐₓ = 312 nm

Les résultats obtenus sont résumés dans le tableau 1 suivant :

| X (% en poids) | DO résiduelle du dibenzoylméthane à 358 nm après 1 heure d'irradiation UVA | DO résiduelle de la triazine à -312 nm après 1 heure d'irradiation UVA |
|---|---|---|
| 0 | 32 ± 4 % | 87 ± 13% |
| 3 | 84 ± 9 % | 98 ± 12% |

## Revendications

1. Composition cosmétique ou dermatologique, à usage topique, **caractérisée par le fait qu'**elle comprend au moins, dans un support cosmétiquement acceptable :
( ) au moins un filtre UV du type dérivé du dibenzoylméthane et
(b) une quantité efficace de 1,1,3-tri-(2-méthyl-4-hydroxy-5-tert-butylphenyl)-butane.

2. Composition **selon la revendication 1**, **caractérisée par le fait qu'**elle comprend au moins, dans un support cosmétiquement acceptable :
au moins un filtre UV du type dérivé de 1,3,5-triazine.

3. Composition selon la revendication 1 ou 2, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

4. Composition selon la revendication 3, **caractérisée par le fait que** le dérivé de dibenzoylméthane est le 4-(ter.-butyl) 4'-méthoxy dibenzoylméthane.

5. Composition selon la revendication 3, **caractérisée par le fait que** le dérivé de dibenzoylméthane est le 4-isopropyl-dibenzoylméthane.

6. Composition selon l'une quelconque des revendications 1 à 5, où le 1,1,3-tri-(2-méthyl-4-hydroxy-5-tert-butylphenyl)-butane est présent à des teneurs allant de 0,1% à 15 % en poids, par rapport au poids total de la composition.

7. Composition selon l'une quelconque des revendications 1 à 6, où le dérivé du dibenzoylméthane est présent à des teneurs allant de 0,1 % à 15 % en poids, par rapport au poids total de la composition.

8. Composition selon l'une quelconque des revendications 2 à 7, où le dérivé de 1,3,5-triazine répond à la formule (1) suivante : 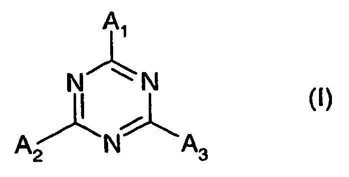
dans laquelle les radicaux A₁, A₂ et A₃, identiques ou différents sont choisis parmi les groupes de formules (II) suivants : 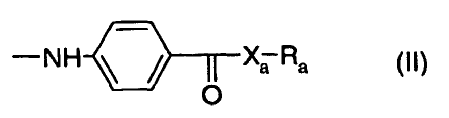
dans lesquels :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 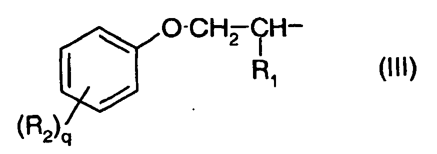 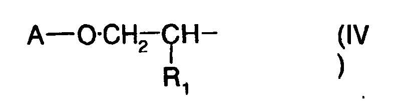 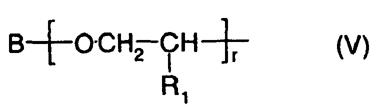
dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

9. Composition selon la revendication 8, où le composé de formule (I) est choisi parmi ceux pour lesquels A₁, A₂ et A₃ sont de formule (II) et présentant l'ensemble des caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) dans lesquelles :
- B est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

10. Composition selon la revendication 8, où le composé de formule (I) est choisi parmi ceux pour lesquels A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) dans lesquelles :
- B est un radical alkyle en C₁-C₄ ;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

11. Composition selon la revendication 10, où le composé de formule (I) est la 2-[(p-(tertiobutylamido)anilino]-4,6-bis-[(p-(2'-éthylhexyl-1'-oxycarbonyl)anilino]-1,3,5-triazine répondant à la formule suivante : 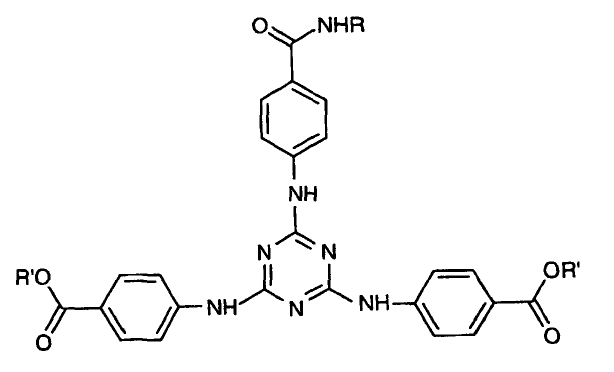
dans laquelle R' désigne un radical éthyl-2 hexyle et R désigne un radical tért-butyle.

12. Composition selon la revendication 8, où le composé de formule (I) est choisi parmi ceux pour lesquels A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

13. Composition selon la revendication 12, où le composé de formule (I) est la 2,4,6-tris[p-(2'-éthylhexyl-1'-oxycarbonyl)anilino]-1,3,5-triazine et répond à la formule suivante : 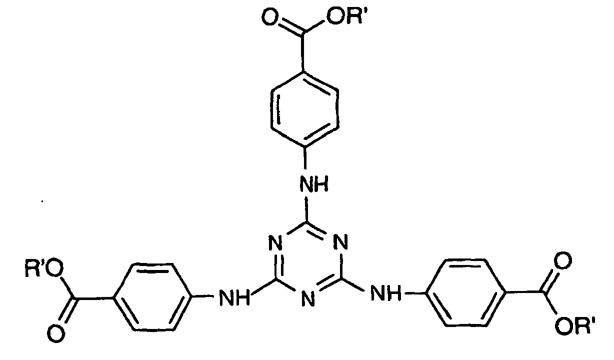
dans laquelle R' désigne un radical 2-éthyl hexyle.

14. Composition selon l'une quelconque des revendications 2 à 13, où le dérivé de 1,3,5-triazine est présent à une teneur allant de 0,1 % à 15 %, par rapport au poids total de la composition.

15. Composition selon l'une quelconque des revendications 1 à 14, **caractérisée par le fait qu'**elle contient en plus d'autres filtres organiques actifs dans l'UV-A et/ou l'UV-B.

16. Composition selon la revendication 15, où le ou les filtres UV organiques complémentaires sont choisis parmi les anthranilates ; les dérivés salicyliques, les dérivés du camphre ; les dérivés de la benzophénone ;les dérivés de triazine autres que ceux de formule (I) ; les dérivés de β,β'-diphénylacrylate, les dérivés de benzotriazole ; les dérivés de benzalmalonate ; les dérivés de benzimidazole ; les imadazolines; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les 4,4-diarylbutadiènes et leurs mélanges.

17. Composition selon la revendication **16**, **caractérisée par le fait que** le ou les filtres UV organiques sont choisis parmi les composés suivants :
- Ethylhexyl Salicylate,
- Octocrylene,
- Phenylbenzimidazole Sulfonic Acid,
- 4-Methylbenzylidene camphor,
- Disodium Phenyl Dibenzimidazole Tetra-sulfonate,
- Terephthalylidene Dicamphor Sulfonic,
- la 2,4,6- tris-(4'amino-benzalmalonate de diisobutyle)-s-triazine
- Anisotriazine,
- Benzophenone-3,
- Benzophenone-4,
- Benzophenone-5,
- le 2-(4-diéthylamino-2-hydroxybenzoyl)-benzoate de n-hexyle
- Methylene bis-Benzotriazolyl Tetramethylbutylphenol,
- Drometrizole Trisiloxane
et leurs mélanges.

18. Composition selon l'une quelconque des revendications 1 à 17, **caractérisée par le fait qu'**elle comprend en outre, des pigments ou des nanopigments d'oxydes métalliques, enrobés ou non.

19. Composition selon la revendication 18, **caractérisée par le fait que** lesdits pigments ou nanopigments sont choisis parmi les oxydes de titane, de zinc, de fer, de zirconium, de cérium et leurs mélanges, enrobés ou non.

20. Composition selon l'une quelconque des revendications 1 à 19, **caractérisée par le fait qu'**elle comprend en outre au moins un agent de bronzage et/ou de brunissage artificiel de la peau.

21. Composition selon l'une quelconque des revendications 1 à 20, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

22. Composition selon l'une quelconque des revendications 1 à 21, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

23. Composition selon l'une quelconque des revendications 1 à 22, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

24. Composition selon l'une quelconque des revendications 1 à 23, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

25. Procédé pour améliorer la stabilité d'au moins un dérivé du dibenzoylméthane tel que défini dans les revendications précédentes vis-à-vis du rayonnement UV, **caractérisé par le fait qu'**il consiste à associer au dit dérivé du dibenzoylméthane une quantité efficace de 1,1,3-tri-(2-méthyl-4-hydroxy-5-tert-butylphenyl)-butane.

26. Utilisation du 1,1,3-tri-(2-méthyl-4-hydroxy-5-tert-butylphenyl)-butane dans la préparation d'une composition cosmétique ou dermatologique comprenant au moins un filtre UV du type dérivé du dibenzoylméthane tel que défini dans les revendications précédentes dans le but de d'améliorer la stabilité vis-à-vis des rayons UV dudit dérivé du dibenzoylméthane.

27. Procédé pour améliorer la stabilité vis-à-vis du rayonnement UV d'au moins un dérivé de 1,3,5-triazine tel que défini dans les revendications précédentes en présence d'un dérivé de dibenzoylméthane tel que défini dans les revendications 2-24, **caractérisé par le fait qu'**il consiste à associer audit dérivé de triazine une quantité efficace de 1,1,3-tri-(2-méthyl-4-hydroxy-5-tert-butylphenyl)-butane.

28. Utilisation du 1,1,3-tri-(2-méthyl-4-hydroxy-5-tert-butylphenyl)-butafie dans la préparation d'une composition cosmétique ou dermatologique comprenant au moins un filtre UV du type dérivé du dibenzoylméthane et au moins un filtre UV du type dérivé de 1,3,5-triazine tels que définis dans les revendications 2-24 dans le but de d'améliorer la stabilité vis-à-vis des rayons UV dudit dérivé de 1,3,5-triazine.

## Patentansprüche

1. Kosmetischer oder dermatologische Zusammensetzung zur topischen Anwendung, **dadurch gekennzeichnet, dass** sie in einem kosmetisch akzeptablen Träger mindestens enthält:
(a) mindestens ein UV-Filter vom Typ eines Derivats von Dibenzoylmethan
und
(b) eine wirksame Menge 1,1,3-Tri-(2-methyl-4-hydroxy-5-tert.-butylphenyl)-butan.

2. Zusammensetzung nach Anspruch 1, **dadurch gekennzeichnet, dass** sie in einem kosmetisch akzeptablen Träger mindestens enthält:
mindestens ein UV-Filter vom Typ eines Derivats von 1,3,5-Triazin.

3. Zusammensetzung nach Anspruch 1 oder 2, **dadurch gekennzeichnet, dass** das Derivat von Dibenzoylmethan ausgewählt ist unter:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

4. Zusammensetzung nach Anspruch 3, **dadurch gekennzeichnet, dass** das Derivat von Dibenzoylmethan 4-(tert.-Butyl)-4'-methoxydibenzoylmethan ist.

5. Zusammensetzung nach Anspruch 3, **dadurch gekennzeichnet, dass** das Derivat von Dibenzoylmethan 4-Isopropyldibenzoylmethan ist.

6. Zusammensetzung nach einem der Ansprüche 1 bis 5, in der das 1,1,3-Tri-(2-methyl-4-hydroxy-5-tert.-butylphenyl)-butan in Mengenanteilen von 0,1 bis 15 Gew.-%, bezogen auf das Gesamtgewicht der Zusammensetzung, vorliegt.

7. Zusammensetzung nach einem der Ansprüche 1 bis 6, in der das Derivat von Dibenzoylmethan in Mengenanteilen von 0,1 bis 15 Gew.-%, bezogen auf das Gesamtgewicht der Zusammensetzung, vorliegt.

8. Zusammensetzung nach einem der Ansprüche 2 bis 7, in der das Derivat von 1,3,5-Triazin die nachstehende Formel (I) aufweist: 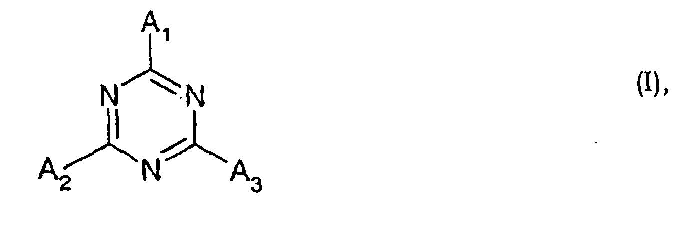 in der die Gruppen A₁, A₂ und A₃, die gleich oder verschieden sind, unter folgenden Gruppen der Formel (II) ausgewählt sind: 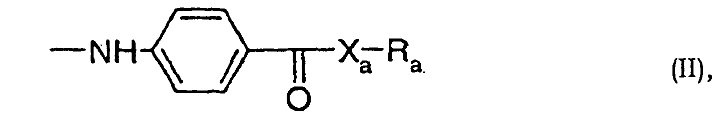
worin
- Xₐ, die gleich oder verschieden sein können, Sauerstoff oder die Gruppe -NH- bedeuten;
- Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Polyoxyethylen-Gruppe, die 1 bis 6 Ethylenoxideinheiten aufweist und deren endständige OH - Gruppe methyliert ist; einer Gruppe der nachstehenden Formeln (III), (IV) oder (V): 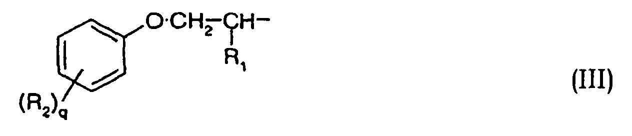 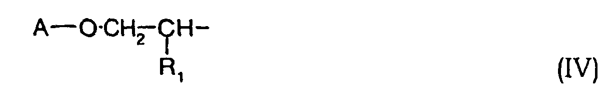 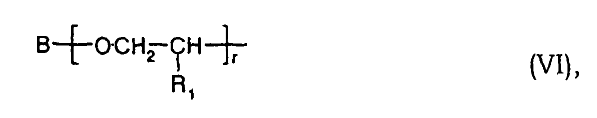
worin:
- R₁ Wasserstoff oder Methyl bedeutet;
- R₂ eine C₁₋₉-Alkylgruppe ist;
- q eine ganze Zahl von 0 bis 3 bedeutet;
- r eine ganze Zahl von 1 bis 10 bedeutet;
- A eine C₄₋₈-Alkylgruppe oder eine C₅₋₈-Cycloalkylgruppe bedeutet;
- B ausgewählt ist unter: einer geradkettigen oder verzweigten C₁₋₈-Alkylgruppe; einer C₅₋₈-Cycloalkylgruppe; einer Arylgruppe, die gegebenenfalls mit einer oder mehreren C₁₋₄-Alkylgruppen substituiert ist.

9. Zusammensetzung nach Anspruch 8, bei der die Verbindung der Formel (I) unter solchen Verbindungen ausgewählt ist, bei denen A₁, A₂ und A₃ die Formel (II) besitzen und bei denen die Gesamtheit folgender Eigenschaften vorliegt:
- eine Gruppe Xₐ-Rₐ bedeutet die Gruppe -NH-Rₐ, wobei Rₐ ausgewählt ist unter: einer C₅₋₁₂-Cycloalkylgruppe, die gegebenenfalls mit einer oder mehreren C₁₋₄-Alkylgruppen substituiert ist; einer Gruppe der Formel (III), (IV) oder (V), worin:
- B eine C₁₋₄-Alkylgruppe bedeutet;
- R₂ Methyl bedeutet;
- die 2 übrigen Gruppen Xₐ-Rₐ die Gruppe -O-Rₐ bedeuten, wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈₋Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁₋₄-Alkylgruppe;
- R₂ Methyl.

10. Zusammensetzung nach Anspruch 8, bei der die Verbindung der Formel (I) unter Verbindungen ausgewählt ist, bei denen A₁, A₂ und A₃ die Formel (II) besitzen und bei denen die Gesamtheit folgender Eigenschaften vorliegt:
- eine oder zwei Gruppen Xₐ-Rₐ bedeuten die Gruppe -NH-Rₐ, wobei Rₐ ausgewählt ist unter: einer geradkettigen oder verzweigten C₁₋₁₈-Alkylglruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V), worin bedeuten:
- B eine C₁₋₄-Alkylgruppe;
- R₂ Methyl;
die übrige Gruppe Xₐ-Rₐ oder die beiden übrigen Gruppen Xₐ-Rₐ bedeuten die Gruppe -O-Rₐ, wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈₋Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁₋₄-Alkylgruppe;
- R₂ Methyl.

11. Zusammensetzung nach Anspruch 10, bei der die Verbindung der Formel (I) 2-[(p-(tert.-Butylamido)-anilino]-4,6-bis[(p-(2'-ethylhexyl-1'-oxycarbonyl)-anilino]-1,3,5-triazin der nachstehenden Formel ist: 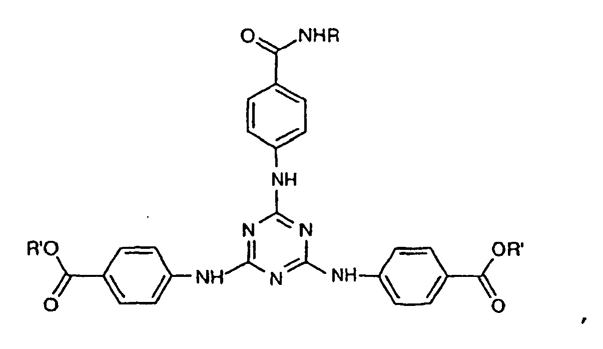 in der R' eine 2-Ethylhexyl-Gruppe und R eine tert.-ButylGruppe bezeichnen.

12. Zusammensetzung nach Anspruch 8, bei der die Verbindung der Formel (I) unter Verbindungen ausgewählt ist, bei denen A₁, A₂ und A₃ die Formel (II) besitzen und bei denen die Gesamtheit folgender Eigenschaften vorliegt:
- Xₐ sind gleich und bedeuten Sauerstoff;
- Rₐ, die gleich oder verschieden sind, bedeuten eine C₆₋₁₂₋Alkylgruppe oder eine Polyoxyethylen-Gruppe, die 1 bis 6 Ethylenoxideinheiten aufweist und deren endständige OH - Gruppe methyliert ist.

13. Zusammensetzung nach Anspruch 12, bei der die Verbindung der Formel (I) 2,4,6-Tris[p-(2'-ethylhexyl-1'-oxycarbonyl)-anilino]-1,3,5-triazin der nachstehenden Formel ist: 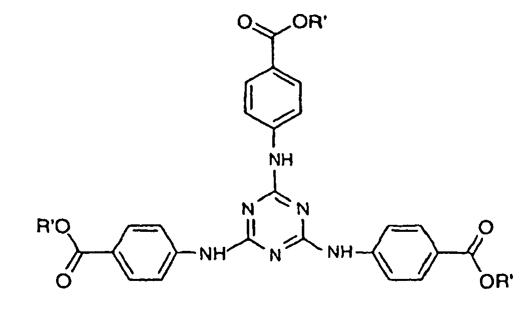 in der R' eine 2-Ethylhexyl-Gruppe bezeichnet.

14. Zusammensetzung nach einem der Ansprüche 2 bis 13, bei der das Derivat von 1,3,5-Triazin in einem Mengenanteil von 0,1 bis 15 %, bezogen auf das Gesamtgewicht der Zusammensetzung, vorliegt.

15. Zusammensetzung nach einem der Ansprüche 1 bis 14, **dadurch gekennzeichnet, dass** sie ferner weitere organische Filter enthält, die im UV-A-Bereich und/ oder im UV-B-Bereich aktiv sind.

16. Zusammensetzung nach Anspruch 15, bei der das oder die zusätzlichen organischen UV-Filter ausgewählt sind unter den Anthranilaten; den Salicylsäurederivaten; den Campherderivaten; den Benzophenon-Derivaten; den Triazin-Derivaten, die von denen der Formel (I) verschieden sind; den Derivaten des β,β-Diphenylacrylats; den Benzotriazol-Derivaten; den Derivaten von Benzalmalonat; den Benzimidazol-Derivaten; den Imidazolinen; den Bis-benzoazolyl-Derivaten; den Derivaten der p-Aminobenzosäure (PABA); den Derivaten von Methylen-bis(hydroxyphenylbenzotriazol); polymeren Filtern und Silicon-Filtern; den von α-Alkylstyrolen abgeleiteten Dimeren; den 4,4-Diarylbutadienen sowie Gemischen dieser Verbindungen.

17. Zusammensetzung nach Anspruch 16, **dadurch gekennzeichnet, dass** das organische UV-Filter oder die organischen UV-Filter unter folgenden Verbindungen ausgewählt sind:
- Ethylhexylsalicylat,
- Octocrylene,
- Phenylbenzimidazolsulfonsäure,
- 4-Methylbenzylidencampher,
- Dinatriumphenyldibenzimidazoltetrasulfonat,
- Terephthalylidendicamphersulfonsäure,
- 2,4,6-Tris(4'-amino-benzaldiisobutylmalonat)-s-triazin,
- Anisotriazine,
- Benzophenone-3,
- Benzophenone-4,
- Benzophenone-5,
- 2-(4-Diethylamino-2-hydroxybenzol)-benzoesäure-n-hexylester,
- Methylene-Bis-Benzotriazolyl Tetramethylbutylphenol,
- Drometrizole Trisiloxane
sowie Gemischen dieser Verbindungen.

18. Zusammensetzung nach einem der Ansprüche 1 bis 17, **dadurch gekennzeichnet, dass** sie ferner Pigmente oder Nanopigmente von Metalloxiden enthält, die gegebenenfalls ummantelt sind.

19. Zusammensetzung nach Anspruch 18, **dadurch gekennzeichnet, dass** die Pigmente oder Nanopigmente ausgewählt sind unter Titanoxid, Zinkoxid, Eisenoxid, Zirconiumoxid, Ceroxid sowie Gemischen dieser Verbindungen, wobei sie gegebenenfalls ummantelt sind.

20. Zusammensetzung nach einem der Ansprüche 1 bis 19, **dadurch gekennzeichnet, dass** sie ferner mindestens ein Mittel zur künstlichen Braunfärbung und/oder künstlichen Bräunung der Haut enthält.

21. Zusammensetzung nach einem der Ansprüche 1 bis 20, **dadurch gekennzeichnet, dass** sie ferner mindestens einen Hilfsstoff enthält, der ausgewählt ist unter Fettkörpern, organischen Lösungsmitteln, ionischen oder nichtionischen Verdickungsmitteln, reizlindernden Mitteln, Antioxidantien, Mitteln gegen freie Radikale, Trübungsmitteln, Stabilisatoren, Emollientien, Siliconen, α-Hydroxysäuren, Antischaummitteln, hydratisierenden Mitteln, Vitaminen, Insektenrepellents, Parfums, Konservierungsmitteln, grenzflächenaktiven Mitteln, entzündungshemmenden Mitteln, Antagonisten gegen Substanz P, Füllstoffen, Polymeren, Treibmitteln, Mitteln zum Alkalischmachen oder zum Ansäuern, Färbemitteln.

22. Zusammensetzung nach einem der Ansprüche 1 bis 21, **dadurch gekennzeichnet, dass** es sich um eine Zusammensetzung zum Schutz der menschlichen Epidermis oder um eine Sonnenschutzzusammensetzung handelt und die Zusammensetzung in Form einer nichtionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stiftes, eines Schaums oder eines Sprays vorliegt.

23. Zusammensetzung nach einem der Ansprüche 1 bis 22, **dadurch gekennzeichnet, dass** es sich um eine Zusammensetzung zum Schminken der Augenbrauen, der Wimpern oder der Haut handelt und die Zusammensetzung in fester oder pastoser Form, wasserfrei oder wasserhaltig, einer Emulsion, einer Suspension oder einer Dispersion vorliegt.

24. Zusammensetzung nach einem der Ansprüche 1 bis 23, **dadurch gekennzeichnet, dass** es sich um eine Zusammensetzung zum Schutz der Haare gegen ultraviolette Strahlung handelt und die Zusammensetzung in Form eines Shampoos, einer Lotion, eines Gels, einer Emulsion oder einer nichtionischen Vesikeldispersion vorliegt.

25. Verfahren zur Verbesserung der Stabilität mindestens eines Derivats von Dibenzoylmethan wie in den vorhergehenden Ansprüchen definiert gegen UV-Strahlung, **dadurch gekennzeichnet, dass** es darin besteht, das Derivat des Dibenzoylmethans mit einer wirksamen Menge 1,1,3-Tri(2-methyl-4-hydroxy-5-tert.-butylphenyl)-butan zu kombinieren.

26. Verwendung von 1,1,3-Tri(2-methyl-4-hydroxy-5-tert.-butylphenyl)-butan bei der Herstellung einer kosmetischen oder dermatologischen Zusammensetzung, die mindestens ein UV-Filter vom Typ eines Derivats von Dibenzoylmethan, wie in den vorhergehenden Ansprüchen definiert, enthält, um die Stabilität des Dibenzoylmethan-Derivats gegen UV-Strahlung zu verbessern.

27. Verfahren zur Verbesserung der Stabilität mindestens eines Derivats von 1,3,5-Triazin, wie in den vorhergehenden Ansprüchen definiert, gegen UV-Strahlung in Gegenwart eines Derivats von Dibenzoylmethan, wie in den Ansprüchen 2 bis 24 definiert, **dadurch gekennzeichnet, dass** es darin besteht, das Triazinderivat mit einer wirksamen Menge 1,1,3-Tri(2-methyl-4-hydroxy-5-tert.-butylphenyl)-butan zu kombinieren.

28. Verwendung von. 1, 1,3-Tri(2-methyl-4-hydroxy-5-tert.-butylphenyl)-butan bei der Herstellung einer kosmetischen oder dermatologischen Zusammensetzung, die mindestens ein UV-Filter vom Typ eines Derivats von Dibenzoylmethan und mindestens ein UV-Filter vom Typ eines Derivats von 1,3,5-Triazin, wie in den Ansprüchen 2 bis 24 definiert, enthält, um die Stabilität des 1,3,5-Triazin-Derivats gegen UV-Strahlung zu verbessern.

## Claims

1. Cosmetic or dermatological composition for topical use, **characterized in that** it comprises at least, in a cosmetically acceptable support:
(a) at least one UV screening agent of the dibenzoylmethane derivative type, and
(b) an effective amount of 1,1,3-tris(2-methyl-4-hydroxy-5-tert-butylphenyl)butane.

2. Composition according to Claim 1, **characterized in that** it comprises at least, in a cosmetically acceptable support:
at least one UV screening agent of the 1,3,5-triazine derivative type.

3. Composition according to Claim 1 or 2, **characterized in that** the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

4. Composition according to Claim 3, **characterized in that** the dibenzoylmethane derivative is 4-(tert-butyl)-4'-methoxydibenzoylmethane.

5. Composition according to Claim 3, **characterized in that** the dibenzoylmethane derivative is 4-isopropyldibenzoylmethane.

6. Composition according to any one of Claims 1 to 5, in which the 1,1,3-tris(2-methyl-4-hydroxy-5-tert-butylphenyl)butane is present in contents ranging from 0.1% to 15% by weight relative to the total weight of the composition.

7. Composition according to any one of Claims 1 to 6, in which the dibenzoylmethane derivative is present in contents ranging from 0.1% to 15% by weight relative to the total weight of the composition.

8. Composition according to any one of Claims 2 to 7, in which the 1,3,5-triazine derivative corresponds to formula (I) below: 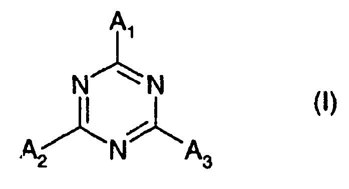 in which the radicals A₁, A₂ and A₃, which may be identical or different, are chosen from the groups of formula (II) below: 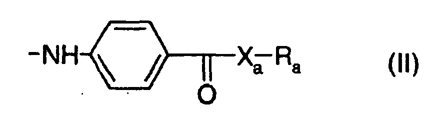
in which:
- Xₐ, which may be identical or different, represent oxygen or an -NH- radical;
- Rₐ, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units, and the terminal OH group of which is methylated; a radical of formula (III), (IV) or (V) below: 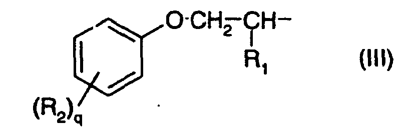 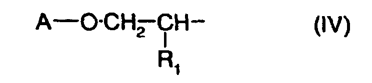 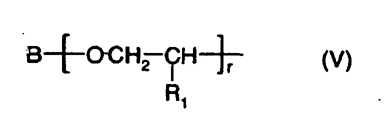
in which:
- R₁ is hydrogen or a methyl radical;
- R₂ is a C₁-C₉ alkyl radical;
- q is an integer from 0 to 3;
- r is an integer from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals.

9. Composition according to Claim 8, in which the compound of formula (I) is chosen from those for which A₁, A₂ and A₃ are of formula (II) and have all of the following characteristics:
- one of the groups Xₐ-Rₐ represents a radical -NH-Rₐ with Rₐ chosen from: a C₅-C₁₂ cycloalkyl radical optionally substituted with one or more C₁-C₄ alkyl radicals; a radical of formula (III), (IV) or (V)
in which:
- B is a C₁-C₄ alkyl radical;
- R₂ is a methyl radical;
- the other 2 groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

10. Composition according to Claim 8, in which the compound of formula (I) is chosen from those for which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (III), (IV) or (V) in which:
- B is a C₁-C₄ alkyl radical;
- R₂ is a methyl radical;
the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

11. Composition according to Claim 10, in which the compound of formula (I) is 2-[(p-(tert-butylamido)anilino]-4,6-bis[(p-(2'ethylhexyl-1'-oxycarbonyl)anilino]-1,3,5-triazine, corresponding to the following formula: 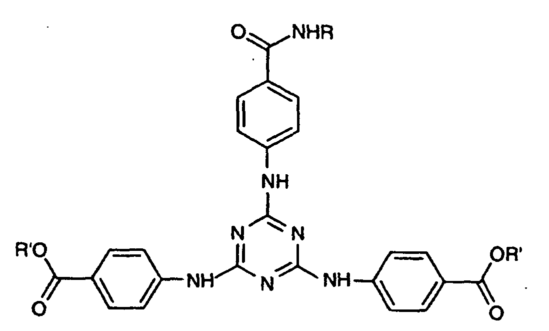
in which R' denotes a 2-ethylhexyl radical and R denotes a tert-butyl radical.

12. Composition according to Claim 8, in which the compound of formula (I) is chosen from those for which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1. to 6 ethylene oxide units, and the terminal OH group of which is methylated.

13. Composition according to Claim 12, in which the compound of formula (I) is 2,4,6-tris[p-(2'-ethylhexyl-1'-oxycarbonyl)anilino]-1,3,5-triazine and corresponds to the following formula: 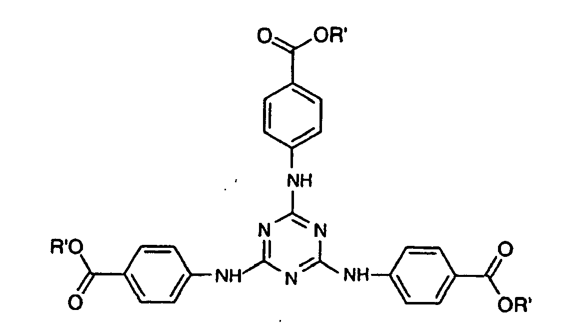
in which R' denotes a 2-ethylhexyl radical.

14. Composition according to any one of Claims 2 to 13, in which the 1,3,5-triazine derivative is present in a content ranging from 0.1% to 15% relative to the total weight of the composition.

15. Composition according to any one of Claims 1 to 14, **characterized in that** it also contains other organic screening agents that are active in the UV-A and/or UV-B range.

16. Composition according to Claim 15, in which the additional organic UV screening agent(s) is (are) chosen from anthranilates; salicylic derivatives; camphor derivatives; benzophenone derivatives; triazine derivatives other than those of formula (I); β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylene-bis(hydroxyphenyl)benzotriazole derivatives; screening polymers and screening silicones; dimers derived from α-alkylstyrene; 4,4-diarylbutadienes, and mixtures thereof.

17. Composition according to Claim 16, **characterized in that** the organic UV screening agent(s) is (are) chosen from the following compounds:
- Ethylhexyl salicylate,
- Octocrylene,
- Phenylbenzimidazolesulphonic acid,
- 4-Methylbenzylidenecamphor,
- Disodium phenyldibenzimidazole tetrasulphonate,
- Terephthalylidenedicamphorsulphonic,
- 2,4,6-tris(diisobutyl 4'-aminobenzalmalonate)-s-triazine,
- Anisotriazine,
- Benzophenone-3,
- Benzophenone-4,
- Benzophenone-5,
- n-hexyl 2-(4-diethylamino-2-hydroxybenzoyl)benzoate,
- Methylenebis(benzotriazolyl)tetramethylbutylphenol,
- Drometrizole trisiloxane
and mixtures thereof.

18. Composition according to any one of Claims 1 to 17, **characterized in that** it also comprises coated or uncoated metal oxide pigments or nanopigments.

19. Composition according to Claim 18, **characterized in that** the said pigments or nanopigments are chosen from titanium oxide, zinc oxide, iron oxide, zirconium oxide and cerium oxide, and mixtures thereof, which may be coated or uncoated.

20. Composition according to any one of Claims 1 to 19, **characterized in that** it also comprises at least one agent for artificially tanning and/or browning the skin.

21. Composition according to any one of Claims 1 to 20, **characterized in that** it also comprises at least one adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellants, fragrances, preserving agents, surfactants, antiinflammatories, substance P antagonists, fillers, polymers, propellants, basifying or acidifying agents, and dyes.

22. Composition according to any one of Claims 1 to 21, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream-gel, a suspension, a dispersion, a powder, a solid tube, a mousse or a spray.

23. Composition according to any one of Claims 1 to 22, **characterized in that** it is a makeup composition for the eyelashes, the eyebrows or the skin and **in that** it is in solid or pasty, anhydrous or aqueous form, or in the form of an emulsion, a suspension or a dispersion.

24. Composition according to any one of Claims 1 to 23, **characterized in that** it is a composition for protecting the hair against ultraviolet rays and **in that** it is in the form of a shampoo, a lotion, a gel, an emulsion or a nonionic vesicular dispersion.

25. Process for improving the stability of at least one dibenzoylmethane derivative as defined in the preceding claims towards UV radiation, **characterized in that** it consists in combining the said dibenzoylmethane derivative with an effective amount of 1,1,3-tris (2-methyl-4-hydroxy-5-tert-butylphenyl)butane.

26. Use of 1,1,3-tris(2-methyl-4-hydroxy-5-tert-butylphenyl)butane in the preparation of a cosmetic or dermatological composition comprising at least one UV screening agent of the dibenzoylmethane derivative type as defined in the preceding claims, with the aim of improving the stability of the said dibenzoylmethane derivative towards UV rays.

27. Process for improving the stability towards UV radiation of at least one 1,3,5-triazine derivative as defined in the preceding claims, in the presence of a dibenzoylmethane derivative as defined in Claims 2-24, **characterized in that** it consists in combining the said triazine derivative with an effective amount of 1,1,3-tris(2-methyl-4-hydroxy-5-tert-butylphenyl)-butane.

28. Use of 1,1,3-tris(2-methyl-4-hydroxy-5-tert-butylphenyl)butane in the preparation of a cosmetic or dermatological composition comprising at least one UV screening agent of the dibenzoylmethane derivative type and at least one UV screening agent of the 1,3,5-triazine derivative type, as defined in Claims 2-24, with the aim of improving the stability of the said 1,3,5-triazine derivative towards UV rays.
